# EUROPÄISCHE PATENTANMELDUNG

(11) **EP 1 832 294 A1**
(43) Veröffentlichungstag der Anmeldung: **12.09.2007**
(21) Anmeldenummer: 07004948.1
(22) Anmeldetag: 09.03.2007
(51) Int. Cl.: A61K 36/185, A61P 17/06, A61K 135/00

(54) **Verwendung von Guajakholz zur Behandlung von Entzündungen der Haut**

(30) Priorität: 09.03.2006 DE 102006011011; 11.09.2006 DE 102006042536
(71) Anmelder: Geske, Gundula, 73277 Owen (DE)
(72) Erfinder: Geske, Gundula, 73277 Owen (DE)
(74) Vertreter: Dey, Michael

(57) **Zusammenfassung**

Die vorliegende Erfindung betrifft die Verwendung eines Extrakts aus Guajakholz zur Herstellung einer pharmazeutischen Zusammensetzung zur Behandlung von Entzündungen der Haut. Weiterhin betrifft die vorliegende Erfindung die Verwendung eines Extrakts aus Guajakholz als Kosmetikum.

## Beschreibung

Die vorliegende Erfindung betrifft die Verwendung eines Extrakts aus Guajakholz zur Herstellung einer pharmazeutischen Zusammensetzung zur Behandlung von Entzündungen der Haut. Weiterhin betrifft die vorliegende Erfindung die Verwendung eines Extrakts aus Guajakholz als Kosmetikum.

Der Guajakbaum gehört zu den in den Tropen und Subtropen verbreiteten Jochblattgewächsen (Zygophyllaceae) und ist auf den westindischen Inseln sowie in den Ländern an der Nordküste Südamerikas beheimatet. Bei den indianischen Stämmen Mittel- und Südamerikas wurde das Holz des Guajakbaums seit langer Zeit volksheilkundlich genutzt. Anfang des 16. Jahrhunderts gelangte das Holz nach Spanien und von dort aus in das übrige Europa, wo es zunächst als "Lignum sanctum" (Heiligenholz) vor allem gegen Syphilis eingesetzt wurde. Im Laufe der Zeit wurde das Guajakholz auch zur Behandlung zahlreicher anderer Erkrankungen, vor allem jedoch bei rheumatischen Erkrankungen, Gelenkentzündungen, Asthma, Tuberkulose und Malaria verabreicht. Anwendungen bei der Behandlung der Pocken brachten dem Tropenholz u.a. den volkstümlichen Namen _{"}Pockholz" ein. Ramos et al. (Arch. Med. Res. 1992; 23, 59-64) beschreiben für einen Extrakt aus *Guajacum coulteri* darüber hinaus einen ausgeprägten hypoglykämischen Effekt.

Guajakholz, bestehend aus dem Kern- sowie dem Splintholz, enthält als Hauptwirkstoff das Guajakharz, das in unregelmäßigen Jahresringen ähnelnden Zonen im Holz eingelagert ist. Das Kernholz enthält bis zu 25 % an Harz, während im Splintholz lediglich 2 bis 3 % an Harz vorliegen. Dieses Harz setzt sich aus verschiedenen Harzsäuren vom Furoguajacintyp (α+β-Guajaconsäure) bzw. Lignantyp (Guajaretsäure und Guajacinsäure), sowie dem Phenol Guajakol zusammen. Weitere Wirkstoffe des Guajakholzes umfassen ätherische Öle (Guajakholzöl), welches vorwiegend aus dem Sesquiterpenalkohol Guajol besteht, Alkaloiden sowie Triterpensaponinen mit dem Aglykon Oleanolsäure.

Während die Inhaltsstoffe des Guajakharzes eine diuretische und diaphoretische Wirkung besitzen, lässt sich die fungizide Wirkung eines Extrakts aus Guajakholz auf die im Holz enthaltenen Saponine zurückführen. Guajakol besitzt seinerseits entzündungshemmende, antiseptische sowie wundheilungsfördernde Eigenschaften. In der Lebensmittelindustrie dient Guajakharz als Antioxidans zur Haltbarmachung vor allem tierischer Fette. Da Guajakharz gleichzeitig ein empfindliches chemisches Reagenz auf Oxidasen und Peroxidasen darstellt, wird es weiterhin zum Blutnachweis in Harn und Stuhl verwendet (Hämoccult-Test). Dieser Nachweis von okkultem Blut beruht auf der Oxidation von α-Guajaconsäure zu chinoidem Guajakblau (Furoguajacinblau).

Obwohl eine Anwendung von Guajakholz bzw. des Guajakharzes im Zusammenhang mit zahlreichen medizinischen Indikationen, insbesondere jedoch im Rahmen der homöopathischen Behandlung von rheumatischen Erkrankungen, beschrieben ist, finden sich in der Literatur keinerlei Studien zur Wirkung eines entsprechenden Extrakts beim Menschen, welche den heutigen Standards genügen würden oder als relevant eingestuft werden könnten. Vor allem jedoch sind der Literatur keinerlei Hinweise auf eine Anwendung eines Extrakts aus Guajakholz zur Behandlung von Entzündungen der Haut zu entnehmen.

GB 2 067 899 A beschreibt kosmetische Zusammensetzungen zur Behandlung der Haut und/oder Haare, welche ein aus Extraktionsrückständen verschiedener Pflanzen erhaltenes Pulver pflanzlichen Ursprungs enthalten. Als eine der Pflanzen, aus welcher nach Extraktion der therapeutisch und/oder kosmetisch wirksamen Stoffe mit Wasser oder organischen Lösungsmitteln ein solcher Extraktionsrückstand erhalten werden kann, ist Guajakholz (Lignum vitae) genannt.

EP 0 265 662 A2 offenbart pharmazeutische Mittel, welche aus zerkleinerten Bestandteilen verschiedener Pflanzen zusammengepresst sind und zur Behandlung verschiedenster Erkrankungen, wie beispielsweise Herzgefäßkrankheiten, Angina pectoris, Psoriasis, allergischer Dermatitis, chronischen Infektionen, Bronchialasthma und Hepatitis, verwendet werden können. Beschrieben ist in diesem Zusammenhang u. a. eine Zusammensetzung, die neben einer Reihe weiterer Bestandteile auch Guajakholz (Guaiacum officinale) in zerkleinerter Form enthält.

JP 10158126 A offenbart ein Haarkosmetikum, welches neben mindestens einer antimikrobiellen Verbindung eine oder mehrere Verbindungen ausgewählt aus der Gruppe bestehend aus Lecithin, Ascorbinsäure, Erythorbinsäure, Guajakharz, Nordihydroguajaretsäure, Gallussäure, einem Ascorbinsäuresalz, einem Erythorbinsäuresalz und einem Nordihydroguajaretsäuresalz enthält.

Infolge des kontinuierlich steigenden Bedarfs, im Hinblick auf eine Behandlung von Entzündungen der Haut auf Produkte natürlichen Ursprungs zurückzugreifen, war es eine Aufgabe der vorliegenden Erfindung, eine Alternative zu synthetischen bzw. anderen natürlichen Produkten mit gleichem oder ähnlichem Wirkspektrum bereitzustellen.

Diese Aufgabe wird erfindungsgemäß gelöst durch die Verwendung eines Extrakts aus Guajakholz, welcher als pharmazeutische Zusammensetzung in geeigneter Formulierung appliziert wird.

Die vorliegende Erfindung betrifft die Verwendung eines Extrakts aus Guajakholz zur Herstellung einer pharmazeutischen Zusammensetzung zur Behandlung von Entzündungen der Haut.

Die Erfindung betrifft weiterhin die Verwendung eines Extrakts aus Guajakholz als Kosmetikum.

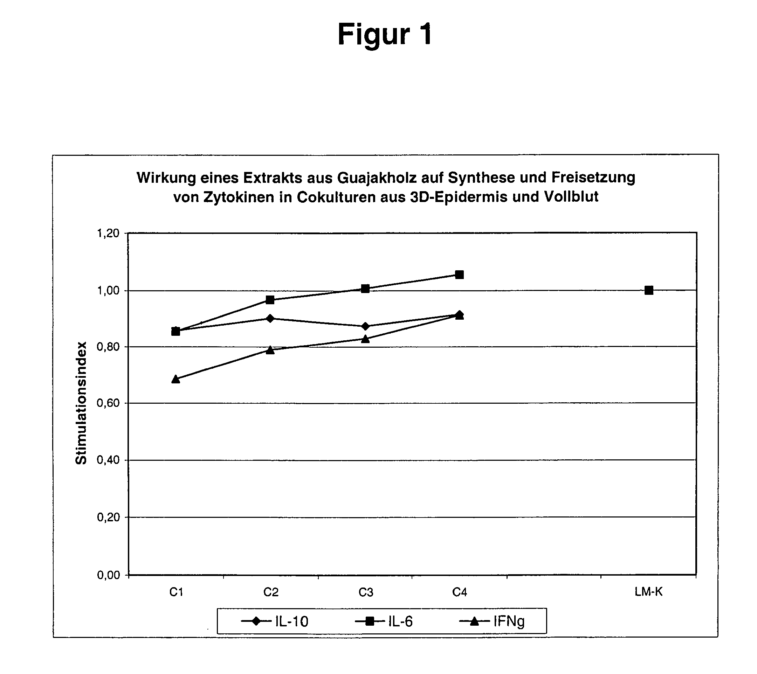

Überraschenderweise wurde im Rahmen der vorliegenden Erfindung festgestellt, dass ein Extrakt aus Guajakholz zur Behandlung von Entzündungen der Haut besonders geeignet ist. Wie anhand von Testergebnissen gezeigt werden konnte, bewirkt ein Extrakt aus Guajakholz nach Resorption der Wirkstoffe über die Haut eine Inhibierung der Freisetzung von Interferon-Gamma (IFNγ), eines für die Entstehung und Aufrechterhaltung entzündlicher Prozesse wichtigen Zytokins (siehe Beispiel in Verbindung mit Figur 1). Die wichtigsten Produzenten dieses Botenstoffs sind T-Helferzellen des Typs 1 (Th1), welche u.a. an der Pathogenese rheumatischer Erkrankungen (Berner et al., J. Rheumatol. 2000, 27, 1128-1135; Vervoordeldonk et al., Curr. Rheumatol. Rep. 2002, 4, 208-217), wie auch an der Chronifizierung der atopischen Dermatitis (Lugovic et al., Int. Arch. Allergy Immunol. 2005, 137, 125-133; Biedermann et al., J. Investig. Dermatol. Symp. Proc. 2004, 9, 5-14) sowie der Pathogenese von Psoriasis (Szegedi et al., Immunol. Lett. 2004, 86, 277-280) wesentlich beteiligt sind.

Erfindungsgemäß wird somit insbesondere die IFNγ-hemmende Aktivität eines Extrakts aus Guajakholz genutzt.

Die Herstellung des Extrakts erfolgt bevorzugt durch Extraktion von Guajakholz mit Wasser, einem aliphatischen Alkohol mit 1 bis 6 Kohlenstoffatomen, oder Kombinationen hiervon. Stärker bevorzugt wird als Auszugsmittel ein Gemisch aus Wasser und einem aliphatischen Alkohol mit 2 bis 4 Kohlenstoffatomen verwendet. Besonders bevorzugt handelt es sich bei dem Auszugsmittel um wässriges Ethanol (70% v/v).

In Übereinstimmung mit der Erfindung umfassen die in vorliegender Anmeldung offenbarten Zusammensetzungen den oben beschriebenen Extrakt aus Guajakholz bevorzugt in einer Menge von 1 bis 50%, stärker bevorzugt von 5 bis 40%, und am stärksten bevorzugt von 10 bis 30% auf Basis des Gesamtgewichts der pharmazeutischen Zusammensetzung.

In einer bevorzugten Ausführungsform umfasst die pharmazeutische Zusammensetzung der vorliegenden Erfindung den Extrakt aus Guajakholz in Kombination mit einem oder mehreren pharmazeutisch annehmbaren Trägern, Verdickungsmitteln, Feuchthaltemitteln oder/und Additiven, wobei der Ausdruck _{"}pharmazeutisch annehmbarer Träger" einen oder mehrere flüssige, halbfeste oder feste Verdünnungsmittel, Füllstoffe oder andere Substanzen bezeichnet, welche für eine Verabreichung an Säuger, einschließlich Menschen, geeignet sind.

Der hier verwendete Ausdruck "pharmazeutisch annehmbar" bezeichnet jegliches nicht-toxische Material, welches die Wirksamkeit der biologischen Aktivität des Wirkstoffes nicht beeinträchtigt. Derartige Materialien können pharmazeutisch annehmbare Konzentrationen an Salzen, Puffern, Konservierungsstoffen oder dergleichen umfassen, wobei es sich bei den Salzen im Falle medizinischer Anwendungen um pharmazeutisch annehmbare Salze handeln sollte. Eine Anwendung von nicht pharmazeutisch annehmbaren Salzen ist dahingehend denkbar, sofern aus derartigen Salzen pharmazeutisch annehmbare Salze hergestellt werden können.

Der Ausdruck "Träger" im Sinne der vorliegenden Erfindung bezeichnet jeglichen organischen oder anorganischen, natürlichen oder synthetischen Stoff, welcher zur Vereinfachung der Applikation mit dem Wirkstoff kombiniert werden kann. Beispiele für derartige Träger umfassen, sind jedoch nicht beschränkt auf, organische oder anorganische Lösungsmittel, Stärke, Laktose, Mannitol, Methylcellulose, Talk, Gelatine, Agar-Agar, Calciumphosphat, Magnesiumstearat, tierische und pflanzliche Fette, höhermolekulare Fettsäuren, oder höhermolekulare Polymere.

Bevorzugte pharmazeutisch annehmbare Träger im Sinne der vorliegenden Erfindung sind flüssige Träger, wie etwa Wasser, wässrige Salzlösungen, nicht-wässrige (wasserfreie) Lösungsmittel, oder Mischungen hieraus. Geeignete nicht-wässrige Lösungsmittel umfassen, sind jedoch nicht beschränkt auf, Ethanol, Propanol, Isopropanol, Butanol, Benzylalkohol, Glycerin, Propylenglykol, Di- oder Tripropylenglykol, Polyethylenglykole, Methylcellosolve, Cellosolve, Morpholine, Dioxan, N,N-Dimethylformamid, Dimethylsulfoxid, Tetrahydrofuran, Phthalate, Adipate oder Ester. In einer besonders bevorzugten Ausführungsform umfasst der verwendete Träger Wasser.

Die erfindungsgemäßen Zusammensetzungen können ferner ein oder mehrere Verdickungsmittel enthalten. Beispiele für Verdickungsmittel, welche im Rahmen der vorliegenden Erfindung verwendet werden können, umfassen insbesondere organische Polymere. Bevorzugt handelt es sich bei dem Verdickungsmittel um einen wasserlöslichen Verdicker wie beispielsweise Cellulosederivate, wobei hydrophil modifizierte Cellulosederivate, wie etwa Hydroxyethylcellulose, Hydroxypropylcellulose, Carboxymethylcellulose, oder/und Hydroxypropylmethylcellulose besonders bevorzugt sind. Besonders geeignete organische Polymere im Sinne der vorliegenden Erfindung sind auch Mucopolysaccharide, wie etwa Hyaluronsäure oder andere Glykosaminoglykane. In einer anderen besonders bevorzugten Ausführungsform kann das Verdickungsmittel eine Polyacrylsäure sein, welche bevorzugt quervernetzt oder/und chemisch modifiziert sein kann, oder ein Salz einer solchen Polyacrylsäure. Bevorzugte Polyacrylsäuren sind quervernetzte Acrylsäurepolymere, z.B. Acrylsäure-Homopolymere, -Copolymere oder -Interpolymere oder Salze solcher Polymere, z.B. Alkali- oder Erdalkalisalze. Hierzu zählen beispielsweise Carbomer-Homopolymere, d.h. hochmolekulare Polymere von Acrylsäure, die mit Polyalkenylethern von Zuckern oder Polyalkoholen, wie etwa Allylsaccharose, Allylpentaerythritol, etc., quervernetzt sind, z.B. Carbopol^{®} 940 NF, 974P NF oder 980 NF. Weiterhin geeignet sind Carbomer-Copolymere, d.h. hochmolekulare Copolymere von Acrylsäure und C₁-C₂₄-Alkylmethacrylaten, quervernetzt mit Polyalkenylethern von Zuckern oder Polyalkoholen, wie etwa Carbopol^{®} 1382, Carbopol^{®} 1342 NF, Carbopol^{®} ETD-2020, Pemulen^{®} TR1 NF und Pemulen^{®} TR2 NF. Als Carbomer-Interpolymere, d.h. Carbomer-Homopolymere oder -Copolymere, die ein heterologes Polymer, z.B. ein Blockcopolymer aus Polyethylenglykol und einem langkettigen, z.B. C₁-C₂₄-Alkylsäureester enthalten, kommen beispielsweise Carbopol^{®} Ultrez 10, 20 oder 21 oder Carbopol^{®} Ultrez 10 NF in Frage.

Erfindungsgemäß kann die pharmazeutische Zusammensetzung der vorliegenden Anmeldung weiterhin ein oder mehrere geeignete Feuchthaltemittel umfassen. Bevorzugte Beispiele für Feuchthaltemittel sind pharmazeutisch verträgliche Polyalkohole, wie etwa Propylenglykol, insbesondere 1,2-Propylenglykol, Pentylenglykol, insbesondere 1,2-Pentandiol, Glycerin oder/und Polyethylenglykol.

Additive im Sinne der vorliegenden Erfindung umfassen beispielsweise Reagenzien zur Einstellung des pH-Werts, Puffer, Verdünnungsmittel, Verarbeitungshilfsmittel wie etwa Emulgatoren, Konservierungsmittel, Stabilisatoren, Antioxidationsmittel, Lichtschutzmittel, Farbstoffe und dergleichen. Als Reagenzien zur Einstellung des pH-Werts werden bevorzugt insbesondere basisch reagierende Verbindungen von Alkali- oder Erdalkalimetallen, wie beispielsweise Hydroxide, Hydrogencarbonate, Carbonate, etc. verwendet, was unter anderem auch metallorganische Verbindungen umfasst. Gleichfalls ist es möglich, gegebenenfalls auch Säuren, umfassend organische und anorganische Säuren, zur Einstellung des pH-Werts einzusetzen. Geeignete Puffer umfassen, sind jedoch nicht beschränkt auf, Essigsäure, Zitronensäure, Weinsäure, Borsäure oder Phosphorsäure in Kombination mit ihren korrespondierenden Basen. Konservierungsstoffe, welche im Rahmen der vorliegenden Erfindung verwendet werden können, umfassen, sind jedoch nicht beschränkt auf, Benzalkoniumchlorid, Chlorbutanol, Thiomersal oder Parabene.

In einer bevorzugten Ausführungsform der Erfindung umfasst die pharmazeutische Zusammensetzung ferner Dexpanthenol, welches eine wichtige Rolle im Hautstoffwechsel einnimmt und im Körper von Säugern zu Pantothensäure (Vitamin B5) metabolisiert wird.

Die erfindungsgemäßen pharmazeutischen Zusammensetzungen sind bevorzugt als Spray, Gel, Creme, Salbe oder Lotion formuliert, wobei eine Formulierung als Creme, Salbe oder Lotion stärker bevorzugt ist. Die Zusammensetzungen können jedoch auch in Form von Aerosolen, wässrigen oder nicht-wässrigen Lösungen, Schäumen, Emulsionen, Suspensionen, oder anderen geeigneten Formulierungen appliziert werden.

Die Verabreichung der erfindungsgemäßen Zusammensetzungen erfolgt bevorzugt topisch. Andere Verabreichungsformen, welche für eine Applikation von Zusammensetzungen im Sinne der vorliegenden Erfindung geeignet sind, sind einem Fachmann auf dem Gebiet der Pharmakologie bzw. Medizin bekannt und umfassen, sind jedoch nicht beschränkt auf, subkutan, intradermal, transdermal, oral, nasal, inhalativ, rektal oder intravenös. Die erfindungsgemäßen Zusammensetzungen werden derart verabreicht, dass pro Verabreichungsdosis bevorzugt eine Menge im Bereich von 10 mg bis 10 g, und besonders bevorzugt eine Menge im Bereich zwischen 100 mg bis 1 g an Trockenextrakt bereitgestellt wird. In einer bevorzugten Ausführungsform handelt es sich bei der verabreichten Menge um eine pharmazeutisch wirksame Menge.

Erfindungsgemäß dienen die in vorliegender Anmeldung beschriebenen Zusammensetzungen der Behandlung von Entzündungen der Haut. Der hier verwendete Ausdruck "Behandlung" bezeichnet dabei eine therapeutische Behandlung, in welcher dem Empfänger eine zur Vorbeugung, Linderung oder Beseitigung der Entzündung oder Allgergie effektive Menge der erfindungsgemäßen Zusammensetzungen verabreicht wird.

Entzündungen der Haut, welche mittels der erfindungsgemäßen Zusammensetzungen behandelbar sind, können durch Allergene, Mikroorganismen oder/und physikalische Reize hervorgerufen sein. In einer bevorzugten Ausführungsform handelt es sich bei den die Entzündung hervorrufenden Allergenen um Fremdallergene, und stärker bevorzugt um Inhalationsallergene, Ingestionsallergene, Kontaktallergene, Injektionsallergene, Invasionsallergene oder/und Depotallergene. Alternativ kann die Entzündung der Haut auch durch Autoallergene hervorgerufen sein.

Inhalationsallergene umfassen, sind jedoch nicht beschränkt auf, Pflanzen- und Gräserpollen, Schimmelpilzsporen, Hausstaubmilbenkot, Tierhaare, Getreide- und Chemikalienstaub, oder Lösungsmitteldämpfe. Ingestionsallergene umfassen, sind jedoch nicht beschränkt auf, Nahrungsmittel (z.B. Milch, Fisch, Obst, Getreide, Nüsse) oder oral einzunehmende Medikamente. Kontaktallergene umfassen, sind jedoch nicht beschränkt auf, Chemikalien, synthetische Stoffe (z.B. Kunststoffe, Desinfektionsmittel, Medikamente), Metalle, Stoffe tierischen Ursprungs (z.B. Wolle, Seide), oder Stoffe pflanzlicher Herkunft (z.B. Harze). Injektionsallergene umfassen, sind jedoch nicht beschränkt auf, Medikamente, Vakzine, Impfseren sowie Insektengift. Invasions- und Depotallergene umfassen, sind jedoch nicht beschränkt auf, Operationsimplantate oder zahnärztliche Wurzelfüllmaterialien.

Der Ausdruck "Mikroorganismen" im Sinne der vorliegenden Erfindung umfasst Bakterien, Pilze, Hefen, Protozoen, Algen und Viren. Bevorzugt handelt es sich bei den die Entzündung hervorrufenden Mikroorganismen um Bakterien, Pilze oder Viren.

Eine Entzündung der Haut kann weiterhin durch physikalische Reize hervorgerufen sein, welche mechanischer, thermischer oder/und chemischer Natur sind. Mechanische Reize umfassen, sind jedoch nicht beschränkt auf, Druck, Verletzungen, Fremdkörpereinwirkung, oder dergleichen. Chemische Reize umfassen, sind jedoch nicht beschränkt auf, Säuren, Laugen, Toxine, entgleiste Enzyme, oder dergleichen. Wärme und Kälte stellen thermische Reize dar, welche Entzündungen der Haut hervorrufen können.

Entzündungen der Haut, welche mittels den erfindungsgemäßen pharmazeutischen Zusammensetzungen behandelt werden können, umfassen, sind jedoch nicht beschränkt auf, Dermatitis und Psoriasis. In einer bevorzugten Ausführungsform handelt es sich bei der zu behandelnden Dermatitis um atopische Dermatitis.

Die vorliegende Erfindung betrifft weiterhin die Verwendung eines Extrakts aus Guajakholz als Kosmetikum, insbesondere als Hautkosmetikum. Das Kosmetikum dient dem Schutz, der Pflege, Reinigung oder/und Verbesserung der Beschaffenheit der Haut.

Die vorliegende Erfinderung wird durch nachfolgendes Beispiel näher erläutert.

### Beispiel

Zur Evaluierung der Wirkung eines Extrakts aus Guajakholz wurden *in vitro-*Untersuchungen an einem für dermatologische Zusammensetzungen relevanten Testsystem vorgenommen, welches eine weitgehende Simulierung der Bedingungen *in vivo* ermöglicht. Hierzu wurde einem Kultursystem aus dreidimensionaler, differenzierter menschlicher Epidermis und Vollblut ein alkoholischer Extrakt aus Guajakholz zugesetzt. Der Aufbau des Testsystems wurde derart gewählt, dass Zellen des Immunsystems und des regulatorisch damit verbundenen Organsystems auf zwei Kulturkompartimente verteilt waren, so dass ein unmittelbarer Zell/Zell-Kontakt ausgeschlossen war, der Austausch von Botenstoffen jedoch stattfinden konnte. Die Inkubation des Kultursystems mit dem Extrakt erfolgte derart, dass die Zellen des Immunsystems lediglich dann mit den aktiven Wirkstoffen des Extrakts in Kontakt kommen konnten, wenn die Wirkstoffe zur Permeation durch die intakte Epidermis befähigt waren.

Figur 1 zeigt die Wirkung eines Extrakts aus Guajakholz auf die Synthese und Freisetzung von Zytokinen aus humanen Leukozyten in Cokulturen aus Epidermis und Vollblut. Hierbei wird eine deutliche Inhibierung der Freisetzung von Interferon-Gamma (IFNγ), eines für die Entstehung und Aufrechterhaltung entzündlicher Prozesse wichtigen Zytokins aus T-Zellen, beobachtet. Darüber hinaus wird die Freisetzung von Interleukin-6 (IL-6) und Interleukin-10 (IL-10) in geringerem Ausmaß inhibiert.

## Patentansprüche

1. Verwendung eines Extrakts aus Guajakholz zur Herstellung einer pharmazeutischen Zusammensetzung zur Behandlung von Entzündungen der Haut.

2. Verwendung nach Anspruch 1, **dadurch gekennzeichnet, dass** die pharmazeutische Zusammensetzung den Extrakt aus Guajakholz in einer Menge von 1 bis 50% auf Basis des Gesamtgewichts der Zusammensetzung enthält.

3. Verwendung nach Anspruch 1 oder 2, **dadurch gekennzeichnet, dass** die Zusammensetzung weiterhin pharmazeutisch annehmbare Träger, Verdickungsmittel, Feuchthaltemittel oder/und Additive umfasst.

4. Verwendung nach Anspruch 3, **dadurch gekennzeichnet, dass** der pharmazeutisch annehmbare Träger Wasser, eine wässrige Salzlösung, ein nicht-wässriges Lösungsmittel, oder Mischungen hieraus umfasst.

5. Verwendung nach Anspruch 3 oder 4, **dadurch gekennzeichnet, dass** das Verdickungsmittel ein organisches Polymer umfasst.

6. Verwendung nach Anspruch 5, **dadurch gekennzeichnet, dass** das organische Polymer ein hydrophil modifiziertes Cellulosederivat, ein Mucopolysaccharid oder/und eine Polyacrylsäure umfasst.

7. Verwendung nach Anspruch 6, **dadurch gekennzeichnet, dass** die Polyacrylsäure eine quervernetzte oder/und chemisch modifizierte Polyacrylsäure umfasst.

8. Verwendung nach einem der Ansprüche 3-7, **dadurch gekennzeichnet, dass** das Feuchthaltemittel Propylenglykol, Pentylenglykol, Glycerin oder/und Polyethylenglykol umfasst.

9. Verwendung nach einem der Ansprüche 1-8, **dadurch gekennzeichnet, dass** die Zusammensetzung weiterhin Dexpanthenol umfasst.

10. Verwendung nach einem der Ansprüche 1-9, **dadurch gekennzeichnet, dass** die pharmazeutische Zusammensetzung als Spray, Gel, Creme, Salbe oder Lotion formuliert ist.

11. Verwendung nach einem der Ansprüche 1-10, **dadurch gekennzeichnet, dass** die pharmazeutische Zusammensetzung für eine topische Verabreichung formuliert ist.

12. Verwendung nach einem der Ansprüche 1-11, **dadurch gekennzeichnet, dass** die Entzündung durch Allergene, Mikroorganismen oder/und physikalische Reize hervorgerufen wird.

13. Verwendung nach Anspruch 12, **dadurch gekennzeichnet, dass** es sich bei den Allergenen um Fremdallergene oder/und Autoallergene handelt.

14. Verwendung nach Anspruch 13, **dadurch gekennzeichnet, dass** es sich bei den Fremdallergenen um Inhalationsallergene, Ingestionsallergene, Kontaktallergene, Injektionsallergene, Invasionsallergene oder/und Depotallergene handelt.

15. Verwendung nach Anspruch 12, **dadurch gekennzeichnet, dass** es sich bei den Mikroorganismen um Bakterien, Pilze oder/und Viren handelt.

16. Verwendung nach Anspruch 12, **dadurch gekennzeichnet, dass** die physikalischen Reize mechanischer, thermischer oder/und chemischer Natur sind.

17. Verwendung nach einem der Ansprüche 1-16, **dadurch gekennzeichnet, dass** es sich bei der Entzündung der Haut um Dermatitis oder/und Psoriasis handelt.

18. Verwendung nach Anspruch 17, **dadurch gekennzeichnet, dass** es sich bei der Dermatitis um atopische Dermatitis handelt.

19. Verwendung eines Extrakts aus Guajakholz zur Behandlung von Entzündungen der Haut.

20. Verfahren zur Behandlung von Entzündungen der Haut, **dadurch gekennzeichnet, dass** einem Patienten eine pharmazeutisch wirksame Menge eines Extrakts aus Guajakholz verabreicht wird.

21. Verwendung eines Extrakts aus Guajakholz als Kosmetikum.
